# FASCICULE DE BREVET EUROPEEN

(11) **EP 3 755 292 B1**
(45) Date de publication et mention de la délivrance du brevet: **30.11.2022**
(21) Numéro de dépôt: 19711976.1
(22) Date de dépôt: 20.02.2019
(51) Int. Cl.: A61F 13/49, A41B 9/04, A61F 13/496

(54) **CULOTTE ANTIFUITE COMPRENANT UN ASSEMBLAGE DE PROTECTION**
DICHTE UNTERHOSE MIT EINER SCHUTZANORDNUNG
LEAKTIGHT UNDERPANTS COMPRISING A PROTECTIVE ASSEMBLY

(30) Priorité: 20.02.2018 FR 1851448
(43) Date de publication de la demande: 30.12.2020
(73) Titulaire: Morter, Wye-Peygn, 75017 Paris (FR); Rychner, Alexandra, 92200 Neuilly-Sur-Seine (FR)
(72) Inventeur: Morter, Wye-Peygn, 75017 Paris (FR); Rychner, Alexandra, 92200 Neuilly-Sur-Seine (FR)
(74) Mandataire: Santarelli
(86) Numéro de dépôt international: PCT/FR2019/050389
(87) Numéro de publication internationale: WO 2019/162615

(56) Documents cités:
- WO-A1-2012/047650
- FR-A1- 3 077 981
- US-A- 5 546 607
- US-A1- 2014 039 432

## Description

La présente invention concerne une culotte antifuite comprenant un assemblage de protection.

De manière générale, la présente invention concerne le domaine des sous-vêtements et plus spécifiquement le domaine des culottes permettant l'absorption de pertes fluidiques.

La présente invention trouve notamment son application, de manière non limitative, dans le domaine des pertes fluidiques chez la femme en période de menstruation ou dans le domaine des pertes urinaires faibles à modérées chez les personnes souffrant d'incontinence urinaire faible à modérée.

Une solution connue est une culotte antifuite comportant un assemblage de protection décrit, en particulier, dans le document WO 2014/022832.

La culotte antifuite présentée dans le document cité comporte un assemblage de protection comprenant au moins une couche absorbante et une couche drainante, les couches absorbante et drainante étant fixées à une portion du bord de chaque ouverture de jambe et à une portion du bord de l'ouverture de taille.

Cependant, la solution décrite dans ce document présente de nombreux inconvénients.

En effet, les formes spécifiques des différentes couches nécessitent la présence de coutures multiples à l'intérieur de la culotte antifuite.

La présente invention vise à proposer une culotte antifuite avec un assemblage de protection plus esthétique en limitant les coutures de fixation visibles de l'extérieur et en limitant dans la mesure du possible les coutures de fixation visibles de l'intérieur.

A cet effet, la présente invention concerne une culotte antifuite selon les alternatives définies dans chacune des revendications 1 à 3.

Grâce à la liaison d'au moins une couche de l'assemblage de protection au corps de tissu au niveau des coutures latérales, les coutures de fixation de l'assemblage de protection sont en partie réalisées sur des parties du corps de tissu comportant déjà des coutures.

Ces coutures de fixation de l'assemblage de protection ne sont pas visibles de l'extérieur car elles sont superposées dans des zones où des coutures sont déjà présentes. Autrement dit, on profite de coutures préexistantes pour réaliser les coutures de fixation au même endroit.

Ainsi, on limite les lignes de coutures additionnelles dans la culotte antifuite. Moins de coutures sont visibles à l'intérieur de la culotte antifuite.

De plus, le confort pour l'utilisateur est amélioré : la culotte antifuite ayant moins de coutures à l'intérieur, les frottements avec la peau sont réduits.

En outre, l'esthétisme est amélioré, les coutures visibles de l'intérieur comme de l'extérieur étant réduites.

Les coutures d'une partie de l'assemblage de protection sont réalisées au niveau de l'ouverture de taille, là où des coutures sont déjà présentes.

Ainsi, ces coutures de fixation de l'assemblage de protection n'ajoutent pas de coutures visibles de l'extérieur de la culotte antifuite.

En limitant la position de la couche absorbante à l'arrière de la culotte et la position de la couche drainante à l'avant de la culotte, on obtient un assemblage de protection moins épais et plus confortable.

En pratique, la couche absorbante ou la couche drainante est fixée au bord avant et au bord arrière délimitant l'ouverture de taille.

Toujours en pratique, la couche absorbante ou la couche drainante a sensiblement les mêmes dimensions que le corps de tissu.

Lorsque la couche absorbante s'étend de l'avant à l'arrière du corps de tissu, l'assemblage de protection absorbe davantage de pertes fluidiques. Ainsi, le pouvoir absorbant de l'assemblage de protection est plus important. Autrement dit, des pertes fluidiques plus importantes peuvent être absorbées.

Lorsque la couche drainante s'étend de l'avant à l'arrière du corps de tissu, la couche drainante évacue davantage les pertes fluidiques vers la couche absorbante. Ainsi, l'utilisateur n'a pas ou peu la peau moite, voire humide. Autrement dit, le confort de l'utilisateur est optimisé.

Dans un autre mode de réalisation avantageux, l'assemblage de protection comprend une couche imperméable, la couche imperméable s'étendant entre la couche absorbante et le corps de tissu. La couche imperméable est fixée à une portion du bord de chaque ouverture de jambe.

La couche imperméable permet d'éviter que des pertes fluidiques trop importantes transpercent le corps de tissu. Ainsi, la couche imperméable permet d'améliorer la fonction absorbante de l'assemblage de protection.

Dans un autre mode de réalisation avantageux, la couche imperméable s'étend dans la partie d'entrejambe et uniquement sur une portion de la partie arrière du corps de tissu.

La couche imperméable empêche la transpiration de passer. Ici, la couche imperméable s'étend uniquement dans une zone utile, en étant limitée à une portion de la partie arrière de la culotte antifuite.

Dans un mode de réalisation avantageux, la couche drainante comporte une couche externe et une doublure, la couche externe et la doublure ayant les mêmes dimensions que le corps de tissu, et la couche absorbante étant fixée à la doublure.

La présence d'une doublure dans l'assemblage de protection sur laquelle la couche absorbante vient se fixer et le fait que la doublure et la couche externe sont de mêmes dimensions permettent de masquer les coutures de fixation à l'intérieur de la culotte antifuite.

Dans un mode de réalisation avantageux, le corps de tissu est formé d'un tissu imperméabilisé.

Un tissu imperméabilisé remplace ou s'ajoute à la couche imperméable de la culotte antifuite. Ainsi, soit l'épaisseur de la culotte antifuite est réduite, soit la fonction imperméable de la culotte antifuite est améliorée.

Dans un autre mode de réalisation avantageux, la couche absorbante est imperméabilisée sur une face en contact avec le corps de tissu.

D'autres particularités et avantages de l'invention apparaîtront encore dans la description ci-après.

D'autres particularités et avantages de l'invention apparaîtront encore dans la description ci-après.

Aux dessins annexés, donnés à titre d'exemples non limitatifs :
- la figure 1a est une vue schématique en perspective d'une culotte antifuite conforme à un mode de réalisation de l'invention ;
- la figure 1b est une vue schématique d'un patron d'une culotte antifuite conforme à un mode de réalisation de l'invention ;
- les figures 2a à 2d sont des vues schématiques des différentes couches d'un assemblage de protection selon un premier mode de réalisation de l'invention ;
- les figures 3a à 3d sont des vues schématiques des différentes couches d'un assemblage de protection selon un deuxième mode de réalisation de l'invention ;
- les figures 4a à 4d sont des vues schématiques des différentes couches d'un assemblage de protection selon un troisième mode de réalisation de l'invention ;
- les figures 5a à 5e sont des vues schématiques des différentes couches d'un assemblage de protection selon un quatrième mode de réalisation de l'invention ; et
- les figures 6a à 6d sont des vues schématiques des différentes couches d'un assemblage de protection selon un cinquième mode de réalisation de l'invention.

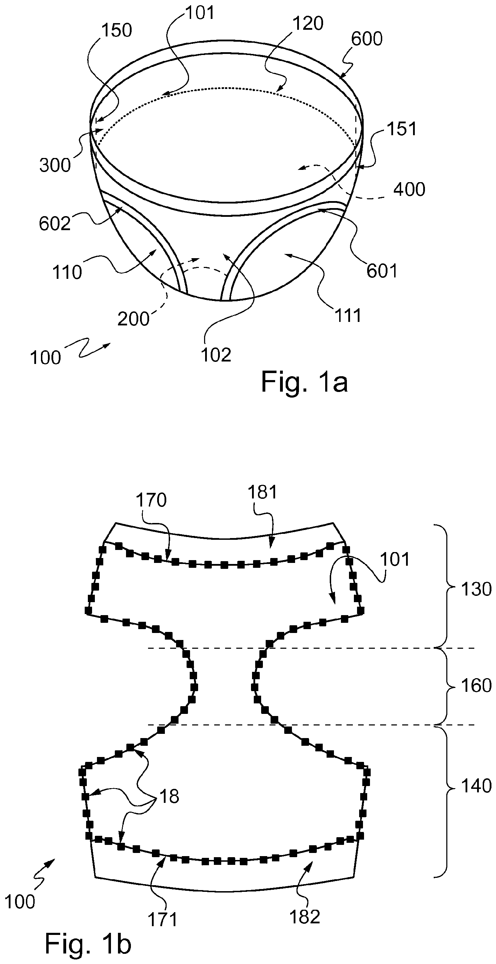

La figure 1a représente une vue schématique en perspective d'une culotte antifuite 100 selon un premier mode de réalisation de l'invention. L'assemblage de protection du premier mode de réalisation est en partie visible ici.

Une culotte antifuite 1 désigne ici tout type de sous-vêtement dans lequel les deux jambes sont séparées, et comme notamment une culotte, un shorty, un string, un boxer, un slip, ...

Typiquement une culotte antifuite 100 est formée d'au moins un corps de tissu. Le corps de tissu est fabriqué en coton, nylon, polyamide tel que le tactel ^{®} ou le meryl ^{®} ou du polyester. Bien entendu, d'autres textiles bien connus de l'homme du métier peuvent être employés.

La culotte antifuite 100 comporte typiquement au moins trois ouvertures. La culotte antifuite 100 est destinée à être portée par une personne, homme, femme, adulte, adolescent ou enfant, et à venir en contact avec le corps de l'individu.

Une culotte antifuite 100 comporte une face intérieure 101 destinée à être en contact avec la peau et une face extérieure 102 opposée à la face intérieure 101 du corps de tissu.

Plus précisément, une culotte antifuite 100 comporte une partie supérieure comportant une ouverture appelée ouverture de taille 120. L'ouverture de taille 120 a un périmètre sensiblement égal à celui du bassin de l'individu portant la culotte antifuite 100.

La culotte antifuite 100 comporte également une partie inférieure comportant deux ouvertures appelées ouvertures de jambes 110, 111. Les ouvertures de jambes 110, 111 sont symétriques par rapport à un plan passant par le milieu de l'ouverture de taille 120.

En référence à la figure 1b, la culotte antifuite 100 comporte aussi une partie avant 130 et une partie arrière 140 comportant respectivement un bord avant 170 et un bord arrière 171 délimitant l'ouverture de taille 120 et destiné à recouvrir une portion de l'abdomen et les fesses de l'individu.

La partie avant 130 et la partie arrière 140 sont fixées l'une à l'autre par des coutures dites coutures latérales 150, 151.

En outre, la culotte antifuite 100 comprend une zone appelée partie d'entrejambe 160. Plus précisément, la partie d'entrejambe 160 est une zone située entre une portion du bord de chaque ouverture de jambe 110, 111 de la culotte antifuite 100.

La culotte antifuite 100 comprend, au moins dans la partie d'entrejambe 160, un ensemble de couches appelé assemblage de protection.

Typiquement, l'assemblage de protection comporte une couche drainante et une couche absorbante.

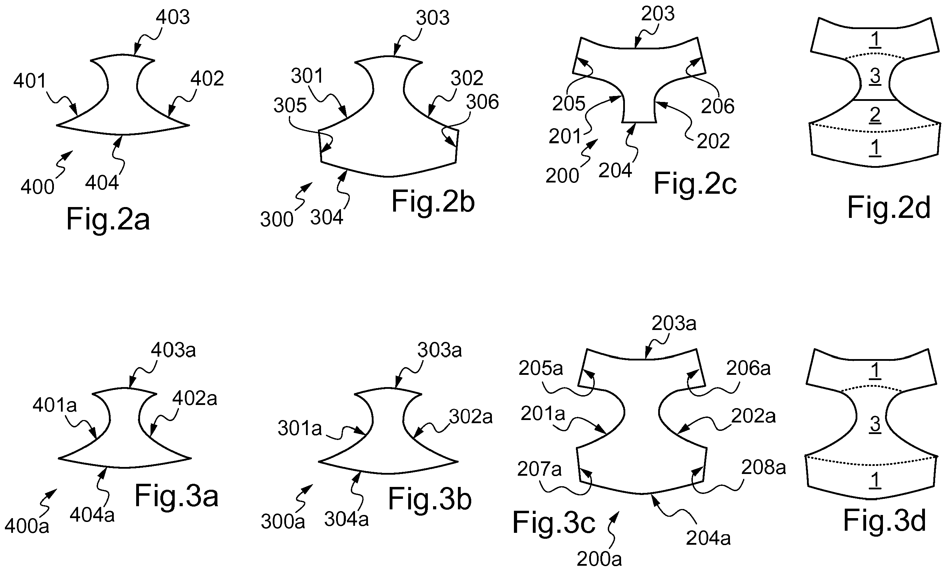

Un exemple de mode de réalisation d'une couche drainante 200 est illustré à la figure 2c et un exemple de mode de réalisation d'une couche absorbante 300 est illustré à la figure 2b.

Typiquement, la couche drainante 200 est fabriquée dans un tissu drainant connu de l'homme du métier, par exemple du polyester ayant un poids moyen de 130g/m2. Cet exemple n'est pas limitatif et d'autres textiles drainants peuvent être employés.

Typiquement, la couche absorbante 300 est fabriquée dans un tissu absorbant connu de l'homme du métier, par exemple du coton flanelle, du coton éponge, du bambou, du chanvre ou de la microfibre. Ces exemples ne sont pas limitatifs et d'autres textiles absorbants peuvent être employés.

Dans un mode de réalisation avantageux, un élastique 600, 601, 602 est fixé le long du bord de l'ouverture de taille 120 et des ouvertures de jambe 110, 111, par exemple, au moyen de points de couture zigzag.

L'élastique 600 de l'ouverture de taille 120 permet de maintenir la culotte antifuite 100 en contact avec la taille.

Les élastiques 601, 602 de chaque ouverture de jambe 110, 111 permettent d'éviter les pertes fluidiques le long des bords des ouvertures de jambe 110, 111.

En référence à la figure 1b, le patron de base de la culotte antifuite 100 est ici formé d'un unique corps de tissu. Les différentes couches de l'assemblage de protection ne sont pas représentées ici.

Dans un mode de réalisation, la culotte antifuite 100 comporte un ruban de dentelle. Le ruban de dentelle comporte deux portions : une portion de dentelle avant 181 et une portion de dentelle arrière 182.

La portion de dentelle avant 181 est fixée au bord avant 170 de l'ouverture de taille 120 et la portion de dentelle arrière est fixée au bord arrière 171 de l'ouverture de taille 120.

Bien entendu, il peut s'agir d'un ruban de tissu de couleur différente de celui du corps de tissu ou d'autres éléments de couture permettant d'embellir la culotte antifuite 100 ou de maintenir la culotte antifuite 100 au corps humain.

Dans un mode de réalisation, la culotte antifuite 100 ne comporte pas de dentelle ou d'autres moyens d'embellissement.

Dans la suite de la demande, cinq modes de réalisation de l'assemblage de protection vont être décrits. L'ordre dans lequel les modes de réalisation vont être présentés ne suppose pas qu'un mode de réalisation est favorisé par rapport à un autre.

Les figures 2a à 2d illustrent un premier mode de réalisation. Plus précisément les figures 2a à 2c présentent respectivement le patron d'une couche imperméable 400, le patron d'une couche absorbante 300 et le patron d'une couche drainante 200 selon un premier mode de réalisation.

La figure 2d présente la superposition des différentes couches de l'assemblage de protection.

En référence à la figure 2c, la couche drainante 200 est représentée.

Lorsque la couche drainante 200 est fixée, elle recouvre tout ou partie de la couche absorbante 300. Autrement dit, la couche drainante 200 comporte une première face en contact, en tout ou partie, avec la couche absorbante 300 et une deuxième face destinée à être en contact avec la peau.

Toujours en référence à la figure 2c, la couche drainante 200 est une pièce de tissu s'étendant sur une partie de l'entrejambe 160 et sur toute la partie avant 130 du corps de tissu.

La couche drainante 200 comporte ici six bords dont trois paires de bords opposés.

Une première paire de bords opposés 205, 206 est fixée tout le long des coutures latérales 150, 151 du corps de tissu de la culotte antifuite 100.

Ainsi, une partie des coutures de fixation de la couche drainante 200 est superposée aux coutures de fixation du corps de tissu de la culotte antifuite 100.

En outre, la couche drainante 200 comporte une deuxième paire de bords opposés 203, 204.

Cette deuxième paire de bords opposés 203, 204 s'étend respectivement vers la partie avant 130 et vers la partie arrière 140 de la culotte absorbante 100.

Plus précisément, l'un des deux bords 203 est fixé tout le long du bord avant 170 délimitant l'ouverture de taille 120. L'autre bord 204 s'étendant vers la partie arrière 140 est un bord droit.

Ainsi, une partie des coutures de fixation de la couche drainante 200 est superposée aux coutures de fixation de la culotte antifuite 100.

Par ailleurs, la couche drainante 200, ayant un bord arrière droit, ne s'étend que dans la partie d'entrejambe 160. Elle ne s'étend que dans la zone utile d'absorption et permet de limiter l'effet couche culotte de la culotte antifuite 100.

En outre, le bord arrière droit 204 de la couche drainante 200 est destiné à être fixé à une face de la couche absorbante 300, non représentée à la figure 2c.

Une troisième paire de bords opposés 201, 202 s'étend le long d'une partie des bords des ouvertures de jambes 110, 111.

Autrement dit, la couche drainante 200 comporte deux bords 201, 202 se superposant à une partie des bords des ouvertures de jambe 110, 111 et fixés aux bords des ouvertures de jambe 110, 111.

Ainsi, une partie des coutures de fixation de la couche drainante 200 est superposée et sont peu ou pas visibles de l'extérieur.

En référence à la figure 2b, la couche absorbante 300 selon le premier mode de réalisation est représentée.

Lorsque la couche absorbante 300 est fixée, elle est disposée en sandwich entre une couche appelée couche imperméable 400, décrite plus loin, et la couche drainante 200. Autrement dit, la couche absorbante 300 comporte une première face destinée à être en contact avec une face de la couche imperméable 400 et une deuxième face destinée à être en contact avec une face de la couche drainante 200.

Toujours en référence à la figure 2b, la couche absorbante 300 est une pièce de tissu s'étendant sur une partie de l'entrejambe 160 et sur toute la partie arrière 140 du corps de tissu.

La couche absorbante 300 comporte ici six bords dont trois paires de bords opposés.

Plus précisément, la couche absorbante 300 comporte une première paire de bords opposés 305, 306 fixés aux coutures latérales 150, 151 du corps de tissu de la culotte absorbante 100.

Ainsi, une partie des coutures de fixation de la couche absorbante 300 est superposée aux coutures de fixation du corps de tissu de la culotte antifuite 100. Ces coutures de fixation ne forment donc pas de coutures supplémentaires sur le sous-vêtement.

La couche absorbante 300 comporte une deuxième paire de bords opposés 303, 304 s'étendant vers la partie avant 130 et vers la partie arrière 140 de la culotte antifuite 100.

Plus précisément, l'un des deux bords 304 est fixé au bord arrière 171 délimitant l'ouverture de taille 120, et ici sur toute la longueur du bord arrière 171 de l'ouverture de taille 120. L'autre bord avant 303 s'étendant vers la partie avant 130 est ici un bord arrondi.

Ainsi, une partie des coutures de fixation de la couche absorbante 300 sont superposées aux coutures de fixation de la culotte absorbante 100.

Par ailleurs, la couche absorbante 300, ayant un bord avant 303 arrondi, ne s'étend que dans la partie d'entrejambe 160, c'est-à-dire dans la zone utile d'absorption et permet de limiter l'effet couche culotte de la culotte antifuite 100.

En outre, le bord avant 303 de la couche absorbante 300 est destiné à être fixé sur une face de la couche drainante 200, non représentée à la figure 2b.

Une troisième paire de bords opposés 301, 302 de la couche absorbante 300 s'étend le long d'une partie des bords des ouvertures de jambes 110, 111.

Autrement dit, la couche absorbante 300 comporte deux bords 301, 302 se superposant et fixés à une partie des bords des ouvertures de jambe 110, 111.

Ainsi, une partie des coutures de fixation de la couche absorbante 300 sont superposées avec les coutures existantes de la culotte antifuite 100 et sont peu ou pas visibles de l'extérieur.

Selon ce premier mode de réalisation, l'assemblage de protection comporte une couche imperméable 400 représentée à la figure 2a.

La couche imperméable 400 s'étend entre le corps de tissu de la culotte absorbante 100 et la couche absorbante 300. La couche imperméable 400 est aussi fixée à une portion du bord de chaque ouverture de jambe 110, 111.

Autrement dit, la couche imperméable 400 comporte une première face destinée à être en contact avec la face interne du corps de tissu de la culotte absorbante 100 et une deuxième face destinée à être en contact avec une face de la couche absorbante 300.

Ici, la couche imperméable 400 est une pièce de tissu s'étendant dans la partie d'entrejambe 160 et uniquement sur une portion de la partie arrière 140 du corps de tissu.

Autrement dit, la couche imperméable 400 comporte ici quatre bords dont deux paires de bords opposés.

Une première paire de bords opposés 401, 402 s'étend le long d'une partie des bords des ouvertures de jambes 110, 111.

Autrement dit, la couche imperméable 400 comporte deux bords 401, 402 se superposant à une partie des bords des ouvertures de jambe 110, 111. Ces bords comportent déjà des coutures nécessaires pour la mise en forme du corps de tissu, notamment pour la fixation des élastiques 601, 602. Ainsi, une partie des coutures de fixation de la couche imperméable 400 sont superposées à des coutures de bord de la culotte antifuite et sont peu ou pas visibles de l'extérieur.

Une deuxième paire de bords opposés avant 403 et arrière 404 est fixée sur la couche absorbante 300, la partie avant et la partie arrière de la couche imperméable 400 s'étendant respectivement vers la partie avant 130 et vers la partie arrière 140 du corps de tissu.

Avantageusement, cette deuxième paire de bords opposés avant 403 et arrière 404 s'étend en forme d'arrondi depuis la partie d'entrejambe 160. Bien entendu, la paire de bords opposés avant 403 et arrière 404 peut prendre une autre forme, par exemple une forme en U ou en V.

Ainsi, la couche imperméable 400 est sensiblement plus étendue que si la deuxième paire de bords opposés avant 403 et arrière 404 a une forme droite permettant de retenir des pertes fluidiques sur des zones sensiblement plus importantes.

Typiquement, la couche imperméable 400 est fabriquée dans un tissu technique hydrophobe, par exemple un tissu technique à base de polyuréthane.

Bien entendu, ce choix de tissu technique hydrophobe n'est pas limitatif. D'autres tissus hydrophobes connus de l'homme du métier peuvent être utilisés.

Tout en étant imperméable, le tissu technique sera choisi de manière à être respirant pour la peau du corps humain.

En référence à la figure 2d, le patron de l'assemblage de protection, selon le premier mode de réalisation, vu sur la face intérieure 101 de la culotte antifuite 100 est représenté avec le nombre de couches superposé par zone.

Lorsque seules les coutures sont visibles sur la face intérieure 101, des courbes en pointillés ont été tracés. Lorsqu'un bord d'une couche et les coutures de fixation sont visibles sur la face intérieure 101 des traits pleins ont été tracés.

Ici, trois coutures sont visibles sur la face intérieure 101. Ainsi, les possibilités de frottement avec la peau sont limitées.

En outre, la capacité d'absorption de l'assemblage de protection est optimisée. En effet, la couche drainante 200 remonte jusqu'au bord avant 170 de l'ouverture de taille 120. La couche absorbante 300 remonte jusqu'au bord arrière 171 de l'ouverture de taille 120.

Par ailleurs, la couche imperméable 400 et la couche absorbante 300 ne couvrent que les zones utiles. Par contre, la couche drainante 200 remonte jusqu'au bord avant 170 de l'ouverture de taille 120. Ainsi, les coutures des parties avant de la couche imperméable 400 et de la couche absorbante 300 sont masquées par la couche drainante 200.

Les figures 3a à 3d illustrent un deuxième mode de réalisation. Plus précisément les figures 3a à 3c présentent respectivement le patron d'une couche imperméable 400a, le patron d'une couche absorbante 300a et le patron d'une couche drainante 200a selon un deuxième mode de réalisation.

La figure 3d présente la superposition des différentes couches de l'assemblage de protection.

En référence à la figure 3c, la couche drainante 200a est représentée.

Lorsque la couche drainante 200a est fixée, elle recouvre entièrement la couche absorbante 300a, non représentée à la figure 3c. Autrement dit, la couche drainante 200a comporte une première face destinée à être en contact avec la couche absorbante 300a et une deuxième face destinée à être en contact avec la peau.

Dans ce mode de réalisation, la couche drainante 200a s'étend au-delà de la couche absorbante 300a.

Toujours en référence à la figure 3c, la couche drainante 200a est une pièce de tissu s'étendant sur toute la partie d'entrejambe 160, sur la partie avant 130 et la partie arrière 140 du corps de tissu.

Autrement dit, la couche drainante 200a a ici sensiblement les mêmes dimensions que le corps de tissu.

Plus précisément, la couche drainante 200a comporte ici huit bords dont quatre paires de bords opposés.

Une première paire de bords opposés 205a, 206a est fixée aux coutures latérales 150, 151 de la partie avant 130 du corps de tissu de la culotte antifuite 100.

Ainsi, une partie des coutures de fixation de la couche drainante 200a est superposée aux coutures de fixation du corps de tissu de la culotte antifuite 100.

De plus, la couche drainante 200a comporte une deuxième paire de bords opposés 207a, 208a destinée à être fixée aux coutures latérales 150, 151 du corps de tissu de la culotte antifuite 100.

Ainsi, une partie des coutures de fixation de la couche drainante 200a est superposée aux coutures de fixation du corps de tissu de la culotte antifuite 100.

En outre, la couche drainante 200a comporte une troisième paire de bords opposés 203a, 204a.

Cette troisième paire de bords opposés 203a, 204a s'étend respectivement vers la partie avant 130 et vers la partie arrière 140 de la culotte antifuite 100 lorsque la couche drainante 200a est positionnée dans la culotte antifuite 100.

Plus précisément, le bord avant 203a et le bord arrière 204a sont destinés à être fixés respectivement tout le long du bord avant 170 et tout le long du bord arrière 171 délimitant l'ouverture de taille 120.

Autrement dit, la couche drainante 200a est destinée à être fixée à la fois au bord avant 130 et au bord arrière 140 délimitant l'ouverture de taille 120.

Ainsi, une partie des coutures de fixation de la couche drainante 200 montée dans la culotte antifuite 100 est superposée aux coutures de fixation de la culotte antifuite 100.

De plus, la couche drainante 200a comporte une quatrième paire de bords opposés 201a, 202a s'étendant le long d'une partie des bords des ouvertures de jambe 110, 111.

Autrement dit, la couche drainante 200a comporte deux bords 201a, 202a se superposant et étant fixés à une partie des bords des ouvertures de jambe 110, 111.

Ainsi, les coutures de fixation de la couche drainante 200a sont peu ou pas visibles de l'extérieur de la culotte antifuite 100.

En référence à la figure 3b, la couche absorbante 300a selon le deuxième mode de réalisation est représentée.

La couche absorbante 300a est une pièce de tissu s'étendant sur une partie de l'entrejambe 160 et sur une portion limitée de la partie arrière 140 du corps de tissu.

La couche absorbante 300a comporte ici quatre bords dont deux paires de bords opposés.

Une première paire de bords opposés 301a, 302a s'étend le long d'une partie des bords des ouvertures de jambes 110, 111.

Autrement dit, la couche absorbante 300a comporte deux premiers bords 301a, 302a se superposant à une partie des bords des ouvertures de jambe 110, 111. Ces premiers bords 301a, 302a comportent déjà des coutures nécessaires pour la mise en forme du corps de tissu, notamment pour la fixation des élastiques.

Ainsi, une partie des coutures de fixation de la couche absorbante 300a est superposée aux bords des ouvertures de jambe 110, 111 et est peu ou pas visible de l'extérieur.

Une deuxième paire de bords opposés avant 303a et arrière 304a est fixée sur la couche drainante 200a.

Selon ce deuxième mode de réalisation, l'assemblage de protection comporte une couche imperméable 400a représentée à la figure 3a.

La couche imperméable 400a est une pièce de tissu s'étendant sur une partie de l'entrejambe 160 et sur une portion de la partie arrière 140 du corps de tissu.

Comme dans le premier mode de réalisation, la couche imperméable 400a est destinée à être superposée entre la couche absorbante 300a et le corps de tissu.

Plus précisément, la couche imperméable 400a est ici une couche ayant sensiblement les mêmes dimensions que la couche absorbante 300a dans le deuxième mode de réalisation.

Toujours comme dans le premier mode de réalisation, la couche imperméable 400a est une pièce de tissu s'étendant dans la partie d'entrejambe 160 et uniquement sur une portion limitée de la partie arrière 140 du corps de tissu.

Plus précisément, la couche imperméable 400a comporte quatre bords dont deux paires de bords opposés.

Une première paire de bords opposés 401a, 402a s'étend le long d'une partie des bords des ouvertures de jambe 110, 111.

Autrement dit, la couche imperméable 400a comporte deux bords fixés à une partie des bords des ouvertures de jambe 110, 111.

Une deuxième paire de bords opposés avant 403a et arrière 404a est fixée respectivement aux bords opposés avant 303a et arrière 304a de la couche absorbante 300a.

En référence à la figure 3d, le patron de l'assemblage de protection, selon le deuxième mode de réalisation, vu sur la face intérieure 101 de la culotte antifuite 100 est représenté avec le nombre de couches superposées par zone et la présence des coutures visibles de l'intérieur, par une courbe en pointillés.

Ici, seules deux coutures sont visibles sur la face intérieure 101. Ainsi, les possibilités de frottement avec la peau sont limitées. Grâce à la couche drainante 200a de mêmes dimensions que le corps de tissu de la culotte antifuite 100, aucun bord d'une couche de l'assemblage de protection n'est apparent sur la face intérieure 101.

Autrement dit, la couche drainante 200a remonte jusqu'au bord avant 170 et arrière 171 de l'ouverture de taille 120. Ainsi, les coutures des parties avant et arrière de la couche imperméable 400a et de la couche absorbante 300a sont entièrement masquées par la seule couche drainante 200a à l'extérieur.

En outre, la capacité d'absorption de l'assemblage de protection est limitée à la zone utile. En effet, la couche absorbante 300a est limitée à la zone utile.

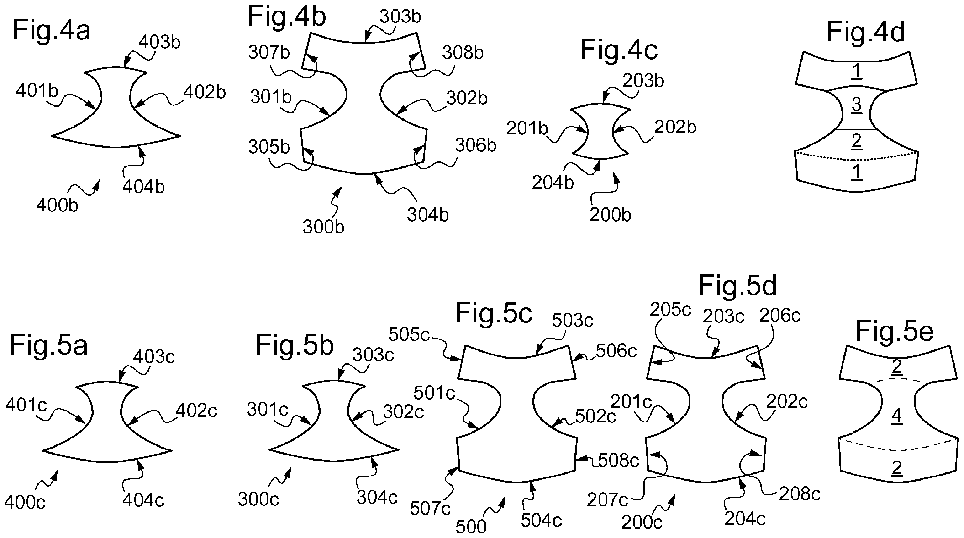

Les figures 4a à 4d illustrent un troisième mode de réalisation. Plus précisément les figures 4a à 4c présentent respectivement le patron d'une couche imperméable 400b, le patron d'une couche absorbante 300b et le patron d'une couche drainante 200b selon un troisième mode de réalisation.

La figure 4d présente la superposition des différentes couches de l'assemblage de protection.

En référence à la figure 4c, la couche drainante 200b est représentée.

Lorsque la couche drainante 200b est fixée à la culotte antifuite 100, elle comporte une première face en contact, en tout ou partie, avec la couche absorbante 300b, décrite plus loin, et une deuxième face destinée à venir en contact avec la peau.

Toujours en référence à la figure 4c, la couche drainante 200b est une pièce de tissu s'étendant uniquement sur une portion de la partie d'entrejambe 160 de la culotte antifuite 100.

Autrement dit, la couche drainante 200b est réduite à la zone minimale utile.

Plus précisément, la couche drainante 200b comporte ici quatre bords dont deux paires de bords opposés.

La couche drainante 200b comporte une première paire de bords opposés 201b, 202b s'étendant le long d'une partie des bords des ouvertures de jambe 110, 111.

Autrement dit, la couche drainante 200b comporte deux bords 201b, 202b se superposant et étant fixés à une partie des bords des ouvertures de jambe 110, 111.

Ainsi, une partie des coutures de fixation de la couche drainante 200b est superposée aux bords des ouvertures de jambe 110, 111 et est peu ou pas visible de l'extérieur.

En outre, la couche drainante 200b comporte une deuxième paire de bords opposés 203b, 204b.

Cette deuxième paire de bords opposés 203b, 204b s'étend respectivement vers la partie avant 130 et vers la partie arrière 140 de la culotte antifuite 100, mais limite la couche drainante 200b à la partie d'entrejambe 160.

En outre, le bord avant 203b et le bord arrière 204b de la deuxième paire de bords opposés de la couche drainante 200b sont fixés à la couche absorbante 300b.

En référence à la figure 4b, la couche absorbante 300b selon le troisième mode de réalisation est représentée.

La couche absorbante 300b est une pièce de tissu s'étendant sur toute la partie d'entrejambe 160, sur la partie avant 130 et la partie arrière 140 du corps de tissu.

Autrement dit, la couche absorbante 300b a ici sensiblement les mêmes dimensions que le corps de tissu.

Plus précisément, la couche absorbante 300b comporte ici huit bords dont quatre paires de bords opposés.

Une première paire de bords opposés 305b, 306b est fixée aux coutures latérales 150, 151 du corps de tissu de la culotte antifuite 100.

Ainsi, lorsque la couche absorbante 300b est fixée au corps de tissu, une partie des coutures de fixation de la couche absorbante 300b est superposée aux coutures de fixation du corps de tissu de la culotte antifuite 100.

De plus, la couche absorbante 300b comporte une deuxième paire de bords opposés 307b, 308b destinés à être fixée aux coutures latérales 150, 151 du corps de tissu de la culotte antifuite 100.

Ainsi, une partie des coutures de fixation de la couche absorbante 300b est superposée aux coutures de fixation du corps de tissu de la culotte antifuite 100.

En outre, la couche absorbante 300b comporte une troisième paire de bords opposés 303b, 304b.

Cette troisième paire de bords opposés 303b, 304b est destinée à s'étendre respectivement vers la partie avant 130 et vers la partie arrière 140 de la culotte antifuite 100.

Plus précisément, le bord avant 303b et le bord arrière 304b sont destinés à être fixés respectivement tout le long du bord avant 170 et tout le long du bord arrière 171 délimitant l'ouverture de taille 120.

Autrement dit, la couche absorbante 300b est destinée à être fixée à la fois au bord avant 170 et au bord arrière 171 délimitant l'ouverture de taille 120.

Ainsi, une partie des coutures de fixation de la couche absorbante 300b sont superposées aux coutures et bords de la culotte antifuite 100.

De plus, la couche absorbante 300b comporte une quatrième paire de bords opposés 301b, 302b s'étendant le long d'une partie des bords des ouvertures de jambe 110, 111.

Autrement dit, la couche absorbante 300b comporte deux bords 301b, 302b se superposant et étant fixés à une partie des bords des ouvertures de jambe 110, 111.

Ainsi, les coutures de fixation de la couche absorbante 300b sont peu ou pas visibles de l'extérieur de la culotte antifuite 100.

En référence à la figure 4a, une couche imperméable 400b, selon ce troisième mode de réalisation, est représentée.

La couche imperméable 400b est une pièce de tissu s'étendant sur une partie de l'entrejambe 160 et sur une portion de la partie arrière 140 du corps de tissu.

Comme dans le premier et le deuxième mode de réalisation, la couche imperméable 400b s'étend entre la couche absorbante 300b et le corps de tissu.

Toujours comme dans le premier et le deuxième mode de réalisation, la couche imperméable 400b est une pièce de tissu s'étendant dans la partie d'entrejambe 160 et uniquement sur une portion de la partie arrière 140 du corps de tissu.

Plus précisément, la couche imperméable 400b comporte quatre bords dont deux paires de bords opposés.

Une première paire de bords opposés 401b, 402b s'étend le long d'une partie des bords des ouvertures de jambe 110, 111.

Autrement dit, la couche imperméable 400b comporte deux bords destinés à être superposés et fixés à une partie des bords des ouvertures de jambe 110, 111.

Une deuxième paire de bords opposés avant 403b et arrière 404b est destinée à être fixée respectivement à la partie avant et à la partie arrière de la couche absorbante 300b, la partie avant et la partie arrière de la couche absorbante 300b s'étendent respectivement sur la partie avant 130 et sur la partie arrière 140 du corps de tissu lorsque la couche imperméable 400b est fixée à la culotte antifuite 100.

En référence à la figure 4d, le patron de l'assemblage de protection, selon le troisième mode de réalisation, vu sur la face intérieure 101 de la culotte antifuite 100 est représenté avec le nombre de couches superposées par zone, la présence des coutures visibles de l'intérieur par une courbe en pointillés et la présence des bords de couche visibles de l'intérieur en trait continu.

Ici, trois coutures seulement sont visibles sur la face intérieure 101. Ainsi, les possibilités de frottement avec la peau sont limitées.

En outre, la capacité d'absorption de l'assemblage de protection est plus grande que dans le premier et le deuxième mode de réalisation. En effet, la couche absorbante 300b remonte jusqu'au bord avant 170 de l'ouverture de taille 120 et jusqu'au bord arrière 171 de l'ouverture de taille 120. La couche absorbante 300b absorbe plus de pertes fluidiques.

Par ailleurs, la couche imperméable 400b et la couche drainante 200b ne couvrent que les zones utiles. Ainsi, dans ce mode de réalisation, la disposition et la forme des couches de l'assemblage de protection sont optimisées par rapport au premier et deuxième mode de réalisation.

Les figures 5a à 5e illustrent un quatrième mode de réalisation. Plus précisément les figures 5a à 5d présentent respectivement le patron d'une couche imperméable 400c, le patron d'une couche absorbante 300c, le patron d'une doublure 500 et le patron d'une couche externe drainante 200c selon un quatrième mode de réalisation.

La figure 5e présente la superposition des différentes couches de l'assemblage de protection.

Ce quatrième mode de réalisation correspond au deuxième mode de réalisation avec une couche supplémentaire appelée doublure 500, la doublure 500 étant destinée à être positionnée entre la couche externe drainante 200c et la couche absorbante 300c.

La couche absorbante 300c et la couche imperméable 400c selon le quatrième mode de réalisation sont sensiblement de même dimension et présentent les mêmes caractéristiques de forme et de fixation au corps de tissu que la couche absorbante 300a et que la couche imperméable 400a selon le deuxième mode de réalisation.

La couche drainante comporte ici la couche externe drainante 200c et la doublure 500. La couche externe drainante 200c et la doublure 500 sont de même dimension et présentent les mêmes caractéristiques de forme et de fixation avec le corps de tissu que la couche drainante 200a du deuxième mode de réalisation.

La couche externe drainante 200c et la doublure 500 ont les mêmes dimensions que le corps de tissu et la couche absorbante 300c est fixée à la doublure 500.

Plus précisément, la doublure 500 est une pièce de tissu s'étendant sur toute la partie d'entrejambe 160, sur la partie avant 130 et la partie arrière 140 du corps de tissu.

Plus précisément, la doublure 500 comporte huit bords dont quatre paires de bords opposés.

Une première paire de bords opposés 505c, 506c est fixée aux coutures latérales 150, 151 du corps de tissu de la culotte antifuite 100.

La première paire de bords opposés 505c, 506c est aussi superposée à la première paire de bords opposés 205c, 206c de la couche externe drainante 200c.

Une deuxième paire de bords opposés 507c, 508c est fixée aux coutures latérales 150, 151 du corps de tissu de la culotte antifuite 100.

La deuxième paire de bords opposés 507c, 508c est aussi fixée à la deuxième paire de bords opposés 207c, 208c de la couche externe drainante 200c.

En outre, la doublure 500 comporte une troisième paire de bords opposés 503c, 504c fixés respectivement au bord avant 170 et au bord arrière 171 délimitant la taille de la culotte antifuite 100.

La troisième paire de bords opposés 503c, 504c est aussi fixée à la troisième paire de bords opposés 203c, 204c de la couche externe drainante 200c.

De plus, la doublure 500 comporte une quatrième paire de bords opposés 501c, 502c s'étendant le long d'une partie des bords des ouvertures de jambes 110, 111.

En référence à la figure 5b, la couche absorbante 300c, selon le quatrième mode de réalisation, est représentée.

La couche absorbante 300c est une pièce de tissu s'étendant sur une partie de l'entrejambe 160 et sur une portion de la partie arrière 140 du corps de tissu.

Ici, la couche absorbante 300c s'étend entre la doublure 500 et la couche imperméable 300c, décrite plus loin.

Comme dans le deuxième mode de réalisation, la couche absorbante 400c est une pièce de tissu s'étendant dans la partie d'entrejambe 160 et uniquement sur une portion de la partie arrière 140 du corps de tissu.

Plus précisément, la couche absorbante 300c comporte quatre bords dont deux paires de bords opposés.

Une première paire de bords opposés 301c, 302c s'étend le long d'une partie des bords des ouvertures de jambes 110, 111.

Autrement dit, la couche absorbante 300c comporte deux bords se superposant et étant fixés à une partie des bords des ouvertures de jambe 110, 111.

Une deuxième paire de bords opposés avant 303c et arrière 304c est fixée respectivement à la partie avant et à la partie arrière de la doublure 500, la partie avant et la partie arrière de la doublure 500 s'étendant respectivement sur la partie avant 130 et sur la partie arrière 140 du corps de tissu lorsque la doublure 500 est fixée à la culotte antifuite 100.

En référence à la figure 5a, une couche imperméable 400c, selon ce quatrième mode de réalisation, est représentée.

La couche imperméable 400c est une pièce de tissu s'étendant sur une partie de l'entrejambe 160 et sur une portion de la partie arrière 140 du corps de tissu.

Comme dans le premier à troisième mode de réalisation, la couche imperméable 400c s'étend entre la couche absorbante 300c et le corps de tissu.

Toujours comme dans le premier à troisième mode de réalisation, la couche imperméable 400c est une pièce de tissu s'étendant dans la partie d'entrejambe 160 et uniquement sur une portion de la partie arrière 140 du corps de tissu.

Plus précisément, la couche imperméable 400c comporte quatre bords dont deux paires de bords opposés.

Une première paire de bords opposés 401c, 402c s'étend le long d'une partie des bords des ouvertures de jambe 110, 111.

Autrement dit, la couche imperméable 400c comporte deux bords 401c, 402c destinés à être fixés à une partie des bords des ouvertures de jambe 110, 111.

Une deuxième paire de bords opposés avant 403c et arrière 404c est fixée respectivement à la couche absorbante 300c à la partie avant et à la partie arrière de la doublure 500.

Ainsi, dans ce quatrième mode de réalisation toutes les coutures de l'assemblage de protection sont masquées par la couche externe drainante 200c ou sont superposées à des régions comportant déjà des coutures comme les ouvertures de jambe 110, 111 et de taille 120.

En référence à la figure 5e, le patron de l'assemblage de protection, selon le quatrième mode de réalisation, vu sur la face intérieure 101 de la culotte antifuite 100, est représenté avec le nombre de couches superposées par zone et la délimitation des différentes couches par une courbe en tirets.

Ici, aucune couture n'est visible de l'intérieur et de l'extérieur de la culotte antifuite 100. Ainsi, les possibilités de frottement avec la peau sont minimisées.

En outre, la capacité d'absorption de l'assemblage de protection est plus grande que dans le premier et deuxième mode de réalisation. En effet, la doublure 500 est une couche supplémentaire drainante, de structure et de matériaux similaires à ceux de la couche externe drainante 200c.

En particulier, la doublure 500 remonte jusqu'au bord avant 170 de l'ouverture de taille 120 et jusqu'au bord arrière 171 de l'ouverture de taille 120. La doublure 500 et la couche externe drainante 200c forment ainsi une couche drainante fixée au bord avant 170 et au bord arrière 171 de l'ouverture de taille 120.

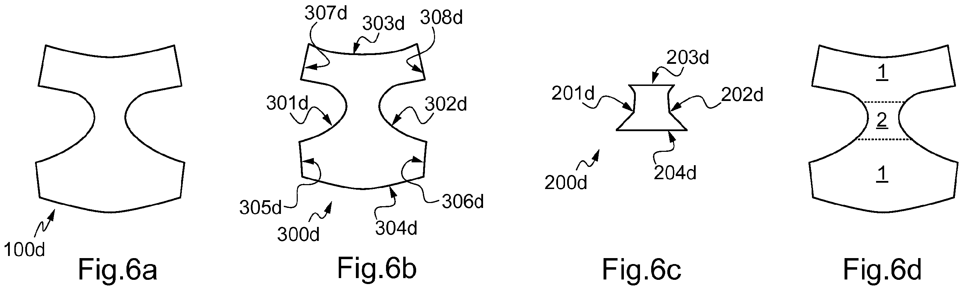

Les figures 6a à 6d illustrent un cinquième mode de réalisation. Plus précisément les figures 6a à 6c présentent respectivement le patron d'un corps de tissu imperméabilisé 100d le patron d'une couche absorbante 300d et le patron d'une couche drainante 200d selon un cinquième mode de réalisation. La figure 6d présente la superposition des différentes couches de l'assemblage de protection.

Ce cinquième mode de réalisation correspond au troisième mode de réalisation, avec un corps de tissu imperméabilisé 100d, sans la couche imperméable 400b.

Autrement dit, la couche drainante 200d dans ce cinquième mode de réalisation a sensiblement les mêmes dimensions et présente les mêmes caractéristiques de forme et de fixation que la couche drainante 200b dans le troisième mode de réalisation. La couche absorbante 300d dans ce cinquième mode de réalisation a sensiblement les mêmes dimensions et présente les mêmes caractéristiques de forme et de fixation que la couche absorbante 300b dans le deuxième mode de réalisation. Le corps de tissu de la culotte antifuite 100 est formé d'un tissu imperméabilisé.

En référence à la figure 6c, la couche drainante 200d est représentée.

Lorsque la couche drainante 200d est fixée, elle comporte une première face en contact, en tout ou partie, avec la couche absorbante 300d, décrite plus loin, et une deuxième face destinée à venir en contact avec la peau.

Toujours en référence à la figure 6c, la couche drainante 200d est une pièce de tissu s'étendant uniquement sur la partie d'entrejambe 160.

Autrement dit, la couche drainante 200d est réduite à la zone minimale utile.

Plus précisément, la couche drainante 200d comporte ici quatre bords dont deux paires de bords opposés.

La couche drainante 200d comporte une première paire de bords opposés 201d, 202d s'étendant le long d'une partie des bords des ouvertures de jambes 110, 111.

Autrement dit, la couche drainante 200d comporte deux bords 201d, 202d superposés et fixés à une partie des bords des ouvertures de jambe 110, 111.

Ainsi, une partie des coutures de fixation de la couche drainante 200d sont peu ou pas visibles de l'extérieur de la culotte antifuite 100d.

En outre, la couche drainante 200d comporte une deuxième paire de bords opposés 203d, 204d.

Cette deuxième paire de bords opposés 203d, 204d s'étend respectivement vers la partie avant 130 et vers la partie arrière 140 de la culotte antifuite 100, mais limite la couche drainante 200d à la partie d'entrejambe 160.

En outre, le bord avant 203d et le bord arrière 204d sont fixés à la couche absorbante 300d.

En référence à la figure 6b, la couche absorbante 300d selon le cinquième mode de réalisation est représentée.

La couche absorbante 300d est une pièce de tissu s'étendant sur toute la partie d'entrejambe 160, sur la partie avant 130 et la partie arrière 140 du corps de tissu.

Autrement dit, la couche absorbante 300d a ici sensiblement les mêmes dimensions que le corps de tissu.

Plus précisément, la couche absorbante 300d comporte ici huit bords dont quatre paires de bords opposés.

Une première paire de bords opposés 305d, 306d est destiné à être fixée aux coutures latérales 150, 151 du corps de tissu de la culotte antifuite 100d.

Ainsi, une partie des coutures de fixation de la couche absorbante 300d est superposée aux coutures de fixation du corps de tissu de la culotte antifuite 100d.

De plus, la couche absorbante 300d comporte une deuxième paire de bords opposés 307d, 308d fixée aux coutures latérales 150, 151 du corps de tissu de la culotte antifuite 100d.

Ainsi, une partie des coutures de fixation de la couche absorbante 300d est destinée être superposée aux coutures de fixation du corps de tissu de la culotte antifuite 100d.

En outre, la couche absorbante 300d comporte une troisième paire de bords opposés 303d, 304d.

Cette troisième paire de bords opposés 303d, 304d s'étend respectivement vers la partie avant 130 et vers la partie arrière 140 de la culotte antifuite 100d.

Plus précisément, le bord avant 303d et le bord arrière 304d sont fixés respectivement tout le long du bord avant 170 et tout le long du bord arrière 171 délimitant l'ouverture de taille 120.

Autrement dit, la couche absorbante 300d est fixée à la fois au bord avant 170 et au bord arrière 171 délimitant l'ouverture de taille 120.

Ainsi, une partie des coutures de fixation de la couche absorbante 300d sont superposées aux coutures et bords de la culotte antifuite 100d.

De plus, la couche absorbante 300d comporte une quatrième paire de bords opposés 301d, 302d s'étendant le long d'une partie des bords des ouvertures de jambe 110, 111.

Autrement dit, la couche absorbante 300d comporte deux bords 301d, 302d se superposant et étant fixés à une partie des bords des ouvertures de jambe 110, 111.

Ainsi, les coutures de fixation de la couche absorbante 300d sont superposées et sont peu ou pas visibles de l'extérieur de la culotte antifuite 100d.

En référence à la figure 6a, le corps de tissu de la culotte absorbante 100d, selon ce cinquième mode de réalisation, est représenté.

Ici, le corps de tissu est formé d'un tissu imperméabilisé. Par exemple, la face interne du corps de tissu peut recevoir un traitement imperméabilisant.

Alternativement, la couche absorbante 300d est elle-même imperméabilisée sur une face en contact avec le corps de tissu.

En référence à la figure 6d, le patron de l'assemblage de protection, selon le cinquième mode de réalisation, vu sur la face intérieure 101 de la culotte antifuite 100d est représenté avec le nombre de couches superposées par zone et la présence des coutures visibles de l'intérieur par une courbe en pointillés.

Ici, seules deux coutures sont visibles sur la face intérieure 101. Ainsi, les possibilités de frottement avec la peau sont limitées.

En outre, la capacité d'absorption de l'assemblage de protection est optimisée. En effet, la couche absorbante 300b remonte jusqu'au bord avant 170 de l'ouverture de taille 120 et jusqu'au bord arrière 171 de l'ouverture de taille 120. La couche absorbante 300b absorbe plus de pertes fluidiques.

Par ailleurs, le nombre de couches de l'assemblage de protection est minimisé par rapport aux autres modes de réalisation. Ainsi, l'assemblage de protection comporte au maximum deux épaisseurs de couches dans la zone utile.

Bien entendu, on peut dans un mode de réalisation alternatif imperméabiliser à la fois la couche absorbante 300d et le corps de tissu de la culotte antifuite 100d.

Bien entendu, pour optimiser l'invisibilité des coutures de fixation, le fil de couture des coutures de fixation pourra être choisi d'une couleur identique ou similaire à celle du corps de tissu ou bien un fil de couture transparent.

## Revendications

1. Culotte antifuite comprenant un corps de tissu avec une ouverture de taille (120) et une paire d'ouvertures de jambes (110, 111), ledit corps de tissu comprenant une partie avant (130) et une partie arrière (140), lesdites parties avant (130) et arrière (140) étant fixées l'une à l'autre par des coutures latérales (150, 151), ladite partie avant (130) du corps de tissu et ladite partie arrière (140) du corps de tissu comportant respectivement un bord avant (170) et un bord arrière (171) délimitant l'ouverture de taille (120), une portion du bord de chaque ouverture de jambe (110, 111) délimitant une partie d'entrejambe (160), ladite culotte antifuite (100) comprenant au moins dans ladite partie d'entrejambe (160) un assemblage de protection, ledit assemblage de protection comprenant au moins une couche absorbante (300) et une couche drainante (200), lesdites couches absorbante (300) et drainante (200) étant fixées à ladite portion dudit bord de chaque ouverture de jambe (110, 111), ladite culotte étant **caractérisée en ce que** ladite couche absorbante (300) et ladite couche drainante (200) sont fixées auxdites coutures latérales (150, 151), et **en ce que** seule ladite couche absorbante (300) parmi ladite couche absorbante (300) et ladite couche drainante (200) est fixée audit bord arrière (171), et seule ladite couche drainante (200) parmi ladite couche absorbante (300) et ladite couche drainante (200) est fixée audit bord avant (170).

2. Culotte antifuite comprenant un corps de tissu avec une ouverture de taille (120) et une paire d'ouvertures de jambes (110, 111), ledit corps de tissu comprenant une partie avant (130) et une partie arrière (140), lesdites parties avant (130) et arrière (140) étant fixées l'une à l'autre par des coutures latérales (150, 151), ladite partie avant (130) du corps de tissu et ladite partie arrière (140) du corps de tissu comportant respectivement un bord avant (170) et un bord arrière (171) délimitant l'ouverture de taille (120), une portion du bord de chaque ouverture de jambe (110, 111) délimitant une partie d'entrejambe (160), ladite culotte antifuite (100) comprenant au moins dans ladite partie d'entrejambe (160) un assemblage de protection, ledit assemblage de protection comprenant au moins une couche absorbante (300a, 300c) et une couche drainante (200a, 200c, 500), lesdites couches absorbante (300a, 300c) et drainante (200a, 200c, 500) étant fixées à ladite portion dudit bord de chaque ouverture de jambe (110, 111), ladite culotte étant **caractérisée en ce que** ladite couche drainante (200a, 200c, 500) est fixée auxdites coutures latérales (150, 151), et **en ce que** seule ladite couche drainante (200a, 200c, 500) parmi ladite couche absorbante (300a, 300c) et ladite couche drainante (200a, 200c, 500) est fixée audit bord avant (170) et audit bord arrière (171) délimitant l'ouverture de taille (120).

3. Culotte antifuite comprenant un corps de tissu avec une ouverture de taille (120) et une paire d'ouvertures de jambes (110, 111), ledit corps de tissu comprenant une partie avant (130) et une partie arrière (140), lesdites parties avant (130) et arrière (140) étant fixées l'une à l'autre par des coutures latérales (150, 151), ladite partie avant (130) du corps de tissu et ladite partie arrière (140) du corps de tissu comportant respectivement un bord avant (170) et un bord arrière (171) délimitant l'ouverture de taille (120), une portion du bord de chaque ouverture de jambe (110, 111) délimitant une partie d'entrejambe (160), ladite culotte antifuite (100) comprenant au moins dans ladite partie d'entrejambe (160) un assemblage de protection, ledit assemblage de protection comprenant au moins une couche absorbante (300b, 300d) et une couche drainante (200b, 200d), lesdites couches absorbante (300b, 300d) et drainante (200b, 200d) étant fixées à ladite portion dudit bord de chaque ouverture de jambe (110, 111), ladite culotte étant **caractérisée en ce que** ladite couche absorbante (300b, 300d) est fixée auxdites coutures latérales (150, 151), et **en ce que** seule ladite couche absorbante (300b, 300d) parmi ladite couche absorbante (300b, 300d) et ladite couche drainante (200b, 200d) est fixée audit bord arrière (171) et audit bord avant (170) délimitant l'ouverture de taille (120).

4. Culotte antifuite conforme à la revendication 3, **caractérisée en ce que** ladite couche absorbante (300b, 300d) a sensiblement les mêmes dimensions que ledit corps de tissu.

5. Culotte antifuite conforme à la revendication 2, **caractérisée en ce que** ladite couche drainante (200a, 200c, 500) a sensiblement les mêmes dimensions que ledit corps de tissu.

6. Culotte antifuite conforme à l'une des revendications précédentes, **caractérisée en ce que** ledit assemblage de protection comprend une couche imperméable (400, 400a, 400b, 400c), ladite couche imperméable (400, 400a, 400b, 400c) s'étendant entre ladite couche absorbante (300, 300a, 300b, 300c) et ledit corps de tissu et **en ce que** ladite couche imperméable (400, 400a, 400b, 400c) est fixée à ladite portion dudit bord de chaque ouverture de jambe (110, 111).

7. Culotte antifuite conforme à la revendication 6, **caractérisée en ce que** ladite couche imperméable (400, 400a, 400b, 400c) s'étend dans ladite partie d'entrejambe (160) et uniquement sur une portion de ladite partie arrière (140) dudit corps de tissu.

8. Culotte antifuite conforme à l'une des revendications 6 ou 7, prises en combinaison avec la revendication 2, **caractérisée en ce que** la couche imperméable (400a, 400c) a sensiblement les mêmes dimensions que la couche absorbante (300a, 300c), la couche drainante (200a, 200c) recouvrant entièrement et s'étendant au-delà de la couche absorbante (300a, 300c).

9. Culotte antifuite conforme à la revendication 3 **caractérisée en ce que** ledit corps de tissu (100d) est formé d'un tissu imperméabilisé.

10. Culotte antifuite conforme à l'une des revendications précédentes, **caractérisée en ce que** ladite couche absorbante (300,300a, 300b, 300c, 300d) est imperméabilisée sur une face en contact avec ledit corps de tissu.

11. Culotte antifuite conforme à l'une des revendications 2, 5 ou 8, **caractérisée en ce que** la couche drainante comporte une couche externe (200c) et une doublure (500), la couche externe (200c) et la doublure (500) ayant les mêmes dimensions que le corps de tissu et la couche absorbante (300c) étant fixée à la doublure (500).

## Patentansprüche

1. Dichte Unterhose, umfassend einen Stoffkörper mit einer Taillenöffnung (120) und einem Paar Beinöffnungen (110, 111), wobei der Stoffkörper einen vorderen Teil (130) und einen hinteren Teil (140) umfasst, wobei der vordere (130) und hintere (140) Teil mit Seitennähten (150, 151) aneinander befestigt sind, wobei der vordere Teil (130) des Stoffkörpers und der hintere Teil (140) des Stoffkörpers jeweils einen vorderen Rand (170) und einen hinteren Rand (171) aufweist, die die Taillenöffnung (120) begrenzen, wobei ein Abschnitt des Randes jeder Beinöffnung (110, 111) einen Schrittteil (160) begrenzt, wobei die dichte Unterhose (100) mindestens im Schrittteil (160) eine Schutzanordnung umfasst, wobei die Schutzanordnung mindestens eine Saugschicht (300) und eine drainierende Schicht (200) umfasst, wobei die Saug- (300) und drainierende (200) Schicht am Abschnitt des Randes jeder Beinöffnung (110, 111) befestigt sind, wobei die Unterhose **dadurch gekennzeichnet ist, dass** die Saugschicht (300) und die drainierende Schicht (200) an den Seitennähten (150, 151) befestigt sind und dass nur die Saugschicht (300) von der Saugschicht (300) und der drainierenden Schicht (200) am hinteren Rand (171) befestigt ist und nur die drainierende Schicht (200) von der Saugschicht (300) und der drainierenden Schicht (200) am vorderen Rand (170) befestigt ist.

2. Dichte Unterhose, umfassend einen Stoffkörper mit einer Taillenöffnung (120) und einem Paar Beinöffnungen (110, 111), wobei der Stoffkörper einen vorderen Teil (130) und einen hinteren Teil (140) umfasst, wobei der vordere (130) und hintere (140) Teil mit Seitennähten (150, 151) aneinander befestigt sind, wobei der vordere Teil (130) des Stoffkörpers und der hintere Teil (140) des Stoffkörpers jeweils einen vorderen Rand (170) und einen hinteren Rand (171) aufweist, die die Taillenöffnung (120) begrenzen, wobei ein Abschnitt des Randes jeder Beinöffnung (110, 111) einen Schrittteil (160) begrenzt, wobei die dichte Unterhose (100) mindestens im Schrittteil (160) eine Schutzanordnung umfasst, wobei die Schutzanordnung mindestens eine Saugschicht (300a, 300c) und eine drainierende Schicht (200a, 200c, 500) umfasst, wobei die Saug- (300a, 300c) und drainierende (200a, 200c, 500) Schicht am Abschnitt des Randes jeder Beinöffnung (110, 111) befestigt sind, wobei die Unterhose **dadurch gekennzeichnet ist, dass** die drainierende Schicht (200a, 200c, 500) an den Seitennähten (150, 151) befestigt ist und dass nur die drainierende Schicht (200a, 200c, 500) von der Saugschicht (300a, 300c) und der drainierenden Schicht (200a, 200c, 500) am vorderen Rand (170) und am hinteren Rand (171) befestigt ist, die die Taillenöffnung (120) begrenzen.

3. Dichte Unterhose, umfassend einen Stoffkörper mit einer Taillenöffnung (120) und einem Paar Beinöffnungen (110, 111), wobei der Stoffkörper einen vorderen Teil (130) und einen hinteren Teil (140) umfasst, wobei der vordere (130) und hintere (140) Teil mit Seitennähten (150, 151) aneinander befestigt sind, wobei der vordere Teil (130) des Stoffkörpers und der hintere Teil (140) des Stoffkörpers jeweils einen vorderen Rand (170) und einen hinteren Rand (171) aufweist, die die Taillenöffnung (120) begrenzen, wobei ein Abschnitt des Randes jeder Beinöffnung (110, 111) einen Schrittteil (160) begrenzt, wobei die dichte Unterhose (100) mindestens im Schrittteil (160) eine Schutzanordnung umfasst, wobei die Schutzanordnung mindestens eine Saugschicht (300b, 300d) und eine drainierende Schicht (200b, 200d) umfasst, wobei die Saug- (300b, 300d) und drainierende (200b, 200d) Schicht am Abschnitt des Randes jeder Beinöffnung (110, 111) befestigt sind, wobei die Unterhose **dadurch gekennzeichnet ist, dass** die Saugschicht (300b, 300d) an den Seitennähten (150, 151) befestigt ist und dass nur die Saugschicht (300b, 300d) von der Saugschicht (300b, 300d) und der drainierenden Schicht (200b, 200d) am hinteren Rand (171) und am vorderen Rand (170) befestigt ist, die die Taillenöffnung (120) begrenzen.

4. Dichte Unterhose nach Anspruch 3, **dadurch gekennzeichnet, dass** die Saugschicht (300b, 300d) etwa die gleichen Abmessungen wie der Stoffkörper hat.

5. Dichte Unterhose nach Anspruch 2, **dadurch gekennzeichnet, dass** die drainierende Schicht (200a, 200c, 500) etwa die gleichen Abmessungen wie der Stoffkörper hat.

6. Dichte Unterhose nach einem der vorangehenden Ansprüche, **dadurch gekennzeichnet, dass** die Schutzanordnung eine undurchlässige Schicht (400, 400a, 400b, 400c) umfasst, wobei sich die undurchlässige Schicht (400, 400a, 400b, 400c) zwischen der Saugschicht (300, 300a, 300b, 300c) und dem Stoffkörper erstreckt und dass die undurchlässige Schicht (400, 400a, 400b, 400c) am Abschnitt des Randes jeder Beinöffnung (110, 111) befestigt ist.

7. Dichte Unterhose nach Anspruch 6, **dadurch gekennzeichnet, dass** sich die undurchlässige Schicht (400, 400a, 400b, 400c) im Schrittteil (160) und nur auf einem Abschnitt des hinteren Teils (140) des Stoffkörpers erstreckt.

8. Dichte Unterhose nach einem der Ansprüche 6 oder 7, herangezogen in Kombination mit Anspruch 2, **dadurch gekennzeichnet, dass** die undurchlässige Schicht (400a, 400c) etwa dieselben Abmessungen wie die Saugschicht (300a, 300c) hat, die drainierende Schicht (200a, 220c) vollständig bedeckt und sich über die Saugschicht (300a, 300c) hinaus erstreckt.

9. Dichte Unterhose nach Anspruch 3, **dadurch gekennzeichnet, dass** der Stoffkörper (100d) aus einem imprägnierten Stoff gebildet ist.

10. Dichte Unterhose nach einem der vorangehenden Ansprüche, **dadurch gekennzeichnet, dass** die Saugschicht (300, 300a, 300b, 300c, 300d) auf einer Fläche im Kontakt mit dem Stoffkörper imprägniert ist.

11. Dichte Unterhose nach einem der Ansprüche 2, 5 oder 8, **dadurch gekennzeichnet, dass** die drainierende Schicht eine Außenschicht (200c) und ein Futter (500) aufweist, wobei die Außenschicht (200c) und das Futter (500) die gleichen Abmessungen wie der Stoffkörper haben und die Saugschicht (300c) am Futter (500) befestigt ist.

## Claims

1. Anti-leak underpants comprising a fabric body having a waist opening (120) and a pair of leg openings (110, 111), said body comprising a front part (130) and a back part (140), said front and back parts (130; 140) being fastened to each other by lateral seams (150, 151), said front part (130) of the fabric body and said back part (140) of the fabric body respectively comprising a front edge (170) and a back edge (171) delimiting the waist opening (120), a portion of the edge of each leg opening (110, 111) delimiting a crotch part (160), said anti-leak underpants (100) comprising at least in said crotch part (160) a protection assemblage, said protection assemblage comprising at least one absorbent layer (300) and a draining layer (200), said absorbent and draining layers (300; 200) being fastened to said portion of said edge of each leg opening (110, 111), said underpants being **characterized in that** said absorbent layer (300) and said draining layer (200) are fastened to said lateral seams (150, 151), and **in that** only said absorbent layer (300) from among said absorbent layer (300) and said draining layer (200) is fastened to said back edge (171), and only said draining layer (200) from among said absorbent layer (300) and said draining layer (200) is fastened to said front edge (170).

2. Anti-leak underpants comprising a fabric body having a waist opening (120) and a pair of leg openings (110, 111), said fabric body comprising a front part (130) and a back part (140), said front and back parts (130; 140) being fastened to each other by lateral seams (150, 151), said front part (130) of the fabric body and said back part (140) of the fabric body respectively comprising a front edge (170) and a back edge (171) delimiting the waist opening (120), a portion of the edge of each leg opening (110, 111) delimiting a crotch part (160), said anti-leak underpants (100) comprising at least in said crotch part (160) a protection assemblage, said protection assemblage comprising at least one absorbent layer (300a, 300c) and a draining layer (200a, 200c, 500), said absorbent and draining layers (300a, 300c; 200a, 200c, 500) being fastened to said portion of said edge of each leg opening (110, 111), said underpants being **characterized in that** said draining layer (200a, 200c, 500) is fastened to said lateral seams (150, 151), and **in that** only said draining layer (200a, 200c, 500) from among said absorbent layer (300a, 300c) and said draining layer (200a, 200c, 500) is fastened to said front edge (170) and to said back edge (171) delimiting the waist opening (120).

3. Anti-leak underpants comprising a fabric body having a waist opening (120) and a pair of leg openings (110, 111), said fabric body comprising a front part (130) and a back part (140), said front and back parts (130; 140) being fastened to each other by lateral seams (150, 151), said front part (130) of the fabric body and said back part (140) of the fabric body respectively comprising a front edge (170) and a back edge (171) delimiting the waist opening (120), a portion of the edge of each leg opening (110, 111) delimiting a crotch part (160), said anti-leak underpants (100) comprising at least in said crotch part (160) a protection assemblage, said protection assemblage comprising at least one absorbent layer (300b , 300d) and a draining layer (200b, 200d), said absorbent and draining layers (300b, 300d; 200b, 200d) being fastened to said portion of said edge of each leg opening (110, 111), said underpants being **characterized in that** said absorbent layer (300b, 300d) is fastened to said lateral seams (150, 151), and **in that** only said absorbent layer (300b, 300d) from among said absorbent layer (300b, 300d) and said draining layer (200b, 200d) is fastened to said back edge (171) and to said front edge (170) delimiting the waist opening (120).

4. Anti-leak underpants according to claim 3, **characterized in that** said absorbent layer (300b, 300d) has substantially the same dimensions as said fabric body.

5. Anti-leak underpants in accordance with claim 2, **characterized in that** said draining layer (200a, 200c, 500) has substantially the same dimensions as said fabric body.

6. Anti-leak underpants in accordance with one of the preceding claims, **characterized in that** said protection assemblage comprises an impermeable layer (400, 400a, 400b, 400c), said impermeable layer (400, 400a, 400b, 400c) extending between said absorbent layer (300, 300a, 300b, 300c) and said fabric body and **in that** said impermeable layer (400, 400a, 400b, 400c) is fastened to said portion of said edge of each leg opening (110, 111).

7. Anti-leak underpants in accordance with claim 6, **characterized in that** said impermeable layer (400, 400a, 400b, 400c) extends in said crotch part (160) and only over a portion of said back part (140) of said fabric body.

8. Anti-leak underpants in accordance with one of claims 6 or 7, taken in combination with claim 2, **characterized in that** the impermeable layer (400a, 400c) has substantially the same dimensions as the absorbent layer (300a, 300c), the draining layer (200a, 200c) fully covering and extending beyond the absorbent layer (300a, 300c).

9. Anti-leak underpants in accordance with claim 3 **characterized in that** said fabric body (100d) is formed from a fabric rendered impermeable.

10. Anti-leak underpants in accordance with one of the preceding claims, **characterized in that** said absorbent layer (300,300a, 300b, 300c, 300d) is rendered impermeable on one face in contact with said fabric body.

11. Anti-leak underpants in accordance with one of claims 2, 5 or 8, **characterized in that** the draining layer comprises an outside layer (200c) and a lining (500), the outside layer (200c) and the lining (500) having the same dimensions as the fabric body and the absorbent layer (300c) being fastened to the lining (500).
